(19) Europäisches Patentamt
European Patent Office
Office européen des brevets

(11) Veröffentlichungsnummer: **0 144 809**
**A2**

(12) # EUROPÄISCHE PATENTANMELDUNG

(21) Anmeldenummer: **84113623.7**

(22) Anmeldetag: **12.11.84**

(51) Int. Cl.⁴: **C 07 F 15/00**
**A 61 K 31/295**

(30) Priorität: **11.11.83 DE 3340806**

(43) Veröffentlichungstag der Anmeldung:
**19.06.85 Patentblatt 85/25**

(84) Benannte Vertragsstaaten:
**AT BE CH DE FR GB IT LI LU NL SE**

(71) Anmelder: **NATEC Institut für naturwissenschaftlich-technische Dienste GmbH**
**Behringstrasse 154**
**D-2000 Hamburg 50(DE)**

(72) Erfinder: **Sagredos, Angelos N. Prof. Dr.**
**Lenhartzstrasse 6**
**D-2000 Hamburg 20(DE)**

(72) Erfinder: **Papageorgiou, Vassilios P. Prof. Dr.**
**Anixeos 32**
**Panorama Thessaloniki(GR)**

(72) Erfinder: **Mellidis, Antonjus S.**
**Karaiskaki 30**
**Thessaloniki(GR)**

(74) Vertreter: **Schulmeyer, Karl-Heinz, Dr.**
**Kieler Strasse 59a**
**D-2087 Hasloh(DE)**

(54) **Platinkomplexverbindungen substituierter 5,8-Dihydroxy-1,4-naphthochinone, Verfahren zu ihre Herstellung und ihre Verwendung.**

(57) Die Erfindung betrifft Platinkomplexe der allgemeinen Formel

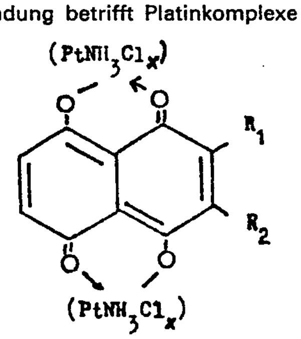

(I)

in der $R_1$ und $R_2$ Alkylreste mit 1 bis 18 C-Atomen oder $R_1$ oder $R_2$ ein Wasserstoffatom und $R_2$ oder $R_1$ einen Alkylrest oder einen Rest

$-CH-CH_2-CH=C(CH_3)_2$
|
$OR_3$

darstellen, wobei $R_3$ ein Wasserstoffatom oder den Rest

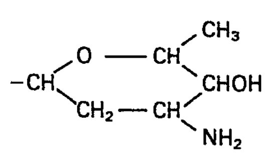

oder einen Acylrest bedeutet und x=1 oder 2 ist.

Diese Verbindungen können hergestellt werden durch Reaktion einer alkoholischen Lösung eines 5,8-Dihydroxy-1,4-naphthochinons der Formel

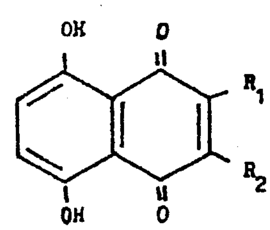

(IV)

./...

EP 0 144 809 A2

in der die Substituenten $R_1$ und $R_2$ die vorstehend angegebenen Bedeutungen haben, in Gegenwart einer konzentrierten wäßbrigen Ammoniaklösung, durch die der pH-Wert auf über 7 eingestellt wird, mit $K_2PtCl_4$ bei Raumtemperatur. Diese Platinkomplexverbindungen zeichnen sich durch eine gute Antitumorwirkung bei geringer Toxizität aus.

**DR. KARL-HEINZ SCHULMEYER**
PATENTANWALT

ZUGELASSEN BEIM EUROPÄISCHEN PATENTAMT
EUROPEAN PATENT ATTORNEY
MANDATAIRE EN BREVETS EUROPÉENS

D-2087 HASLOH BEI HAMBURG
KIELER STRASSE 59 a
TELEFON (0 41 06) 6 64 93
TELEX 21 64 213 watc d

(1032)

Professor
Dr. Angelos N. Sagredos
Lenhartzstraße 6
2000 Hamburg 20

Oktober 1984

# Platinkomplexverbindungen substituierter 5,8-Dihydroxy-1,4-naphthochinone, Verfahren zu ihrer Herstellung und ihre Verwendung

Die Erfindung betrifft Platinkomplexverbindungen von substituierten 5,8-Dihydroxy-1,4-naphthochinonen sowie ein Verfahren zu ihrer Herstellung und ihre Verwendung als Wirkstoffe mit Antitumorwirkung.

Es ist bekannt, daß eine Reihe von Platinverbindungen, in erster Linie das unter der Kurzbezeichnung cis-DDP klinisch angewendete cis-Dichloro-diamino-platin(II), neben allgemein toxischer sowie bakterizider, viruzider und immunosuppressiver Wirkung auch Antitumor-Wirkung hat (Rosenberg, Naturwissenschaften 1973, 399-406). Auch ist bekannt, daß Komplexe dieser quadratisch planaren Platinverbindung mit Anthrachinonderivaten, wie Chinizarin und Doxorubizin, das cis-DDP im Tierkörper nur langsam abgeben, wodurch dessen Toxizität stark herabgesetzt wird. Während vom freien cis-DDP bereits mehr als 7 mg/kg Maus toxisch wirken, können von den genannten Komplexverbindungen noch Dosen von 20 mg cis-DDP/kg Maus appliziert werden, wodurch

sowohl eine deutliche Erhöhung der durchschnittlichen Überlebensdauer als auch eine Steigerung der Heilungsfälle erzielt werden können. Im Zuge der Prüfung der pharmakologischen Wirksamkeit der oben genannten Platin-Anthrachinon-Komplexe wurden zwecks Aufklärung der chemischen Bindungsverhältnisse auch einige relativ einfache α-Hydroxychinone mit Platin komplexiert, aber weder in vitro noch in vivo auf Tumor- oder andere pharmakologische Wirksamkeit geprüft (Yolles et al., ACS-Symposium 1982, 233-241). Bei diesen nur zur Strukturaufklärung hergestellten Naphthochinonkomplexen handelt es sich um

I) 5-Hydroxy-1,4-naphthochinon / cis-DDP-Komplex
II) 5,8-Dihydroxy-1,4-naphthochinon / cis-DDP-Komplex

Es wurde nun gefunden, daß zwei weitere, bisher nicht bekannte Gruppen von Komplexverbindungen von Platin mit 5,8-Dihydroxy-1,4-naphthochinonderivaten eine noch stärkere Antitumor-Wirkung bei geringerer Toxizität zeigen als die bekannten Platinkomplexverbindungen.

Dementsprechend betrifft vorliegende Erfindung Platinkomplexverbindungen der allgemeinen Formel

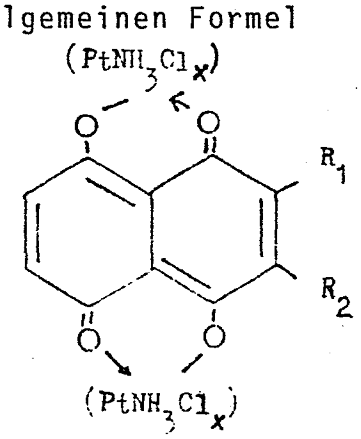

(I)

in der $R_1$ und $R_2$, die gleich oder verschieden sein können, einen Alkylrest mit 1 bis 18 C-Atomen, vorzugsweise mit 1 bis 6 C-Atomen, oder $R_1$ oder $R_2$ ein Wasserstoffatom und $R_2$ oder $R_1$ einen Alkylrest mit 1 bis 18

C-Atomen, vorzugsweise mit 1 bis 6 C-Atomen, oder einen Rest $-\underset{OR_3}{CH}-CH_2-CH=C(CH_3)_2$ darstellen, wobei $R_3$ ein Wasserstoffatom oder den Rest

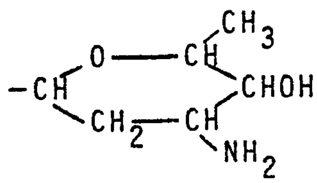 oder einen Acylrest, vorzugsweise den der Angelikasäure $CH_3-CH=C(CH_3)-\overset{O}{\overset{\|}{C}}-$ , bedeutet, und x = 1 oder 2 ist.

Bei den erfindungsgemäßen Platinkomplexverbindungen handelt es sich einerseits um Komplexverbindungen der Formel

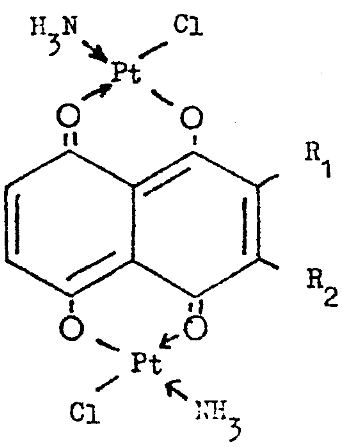

(II)

in denen $R_1$ und $R_2$ die oben angegebenen Bedeutungen haben und x = 1 ist, sowie andererseits um Komplexverbindungen mit gegenüber der Formel II verdoppeltem Chlorgehalt, d.h. x = 2, mit sonst gegenüber der Formel II unveränderter Struktur. Diese Komplexverbindungen lassen sich daher durch die folgende tautomere Formel wiedergeben:

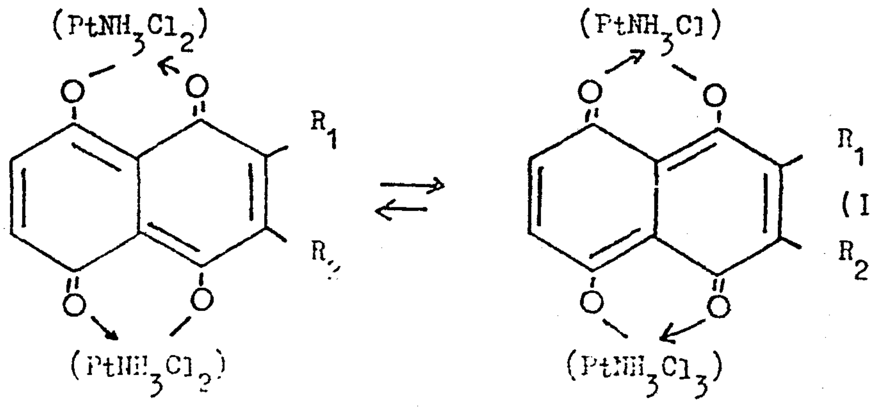

(III)

Es wurde nämlich durch analytische Untersuchungen sichergestellt, daß der organische Teil der Komplexverbindungen der Formel III mit dem organischen Teil der entsprechenden Verbindungen der Formel II völlig identisch ist. Aus den Komplexverbindungen der Formeln II und III lassen sich nämlich ohne Schwierigkeiten die entsprechenden substituierten Dihydroxynaphthochinone durch Hydrolyse abspalten, die mit der eingesetzten organischen Ausgangsverbindung identisch sind.

Die beiden Arten von Komplexverbindungen (II) und (III) können zwar jede für sich in reiner Form dargestellt und erhalten werden, z.B. indem die in den nachstehenden Ausführungsbeispielen genannten Bedingungen genau eingehalten werden. In der Regel fallen sie jedoch nebeneinander an, wenn nicht die nachstehend geschilderten Maßnahmen eingehalten werden.

Das erfindungsgemäße Herstellungsverfahren geht sowohl für die Herstellung der Verbindungen nach Formel II als auch der der Formel III vom entsprechenden Naphthochinonderivat

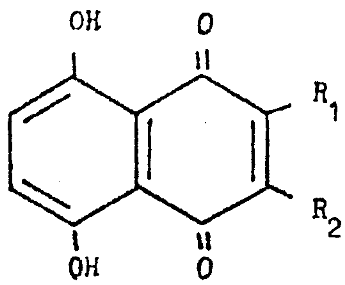

(IV)

aus, wobei die Substituenten $R_1$ bis $R_3$ die vorstehend angegebenen Bedeutungen haben.

Das Verfahren zur Herstellung der erfindungsgemäßen Platinkomplexverbindungen der Formel I wird in der Weise durchgeführt, daß eine alkoholische Lösung eines 5,8-Dihydroxy-1,4-naphthochinons der Formel IV durch Zugabe konzentrierter wäßriger Ammoniaklösung auf einen pH-Wert oberhalb 7 eingestellt und danach unter beständigem Rühren mit einer wäßrigen Lösung von $K_2PtCl_4$ bei Raumtemperatur umgesetzt wird, wobei die Reaktionsmischung mehrere Stunden lang ständig weitergerührt wird. Sämtliche Verfahrensstufen werden vorzugsweise bei Zimmertemperatur durchgeführt. Dabei tritt Komplexbildung unter Umlagerung der Doppelbindungen im Doppelringsystem ein, wobei sich in der Regel Komplexverbindungen der Formel I mit x=1 und x=2 nebeneinander bilden. Durch die Einhaltung bestimmter, relativ enger pH-Wertbereiche während der Umsetzung ist es aber möglich, die Reaktion zugunsten der Bildung von Platinkomplexverbindungen der Formel I mit x=1 oder mit x=2 zu lenken. Erfolgt die Umsetzung eines substituierten 5,8-Dihydroxy-1,4-naphthochinons (IV) mit einer wäßrigen $K_2PtCl_4$-Lösung vorzugsweise bei einem pH-Wert von 8 bis 8,5, dann werden im wesentlichen Platinkomplexverbindungen der Formel II (x=1) erhalten, erfolgt die Umsetzung vorzugsweise bei einem pH-Wert von über 10, dann bilden sich

im wesentlichen Platinkomplexverbindungen der Formel III (x=2).

Die gebildeten Platinkomplexverbindungen fallen als dunkles Pulver aus, das in an sich bekannter Weise abgetrennt und gereinigt werden kann, beispielsweise durch Abfiltrieren und anschließendes Waschen nacheinander mit Wasser, Aceton und einem Ether, z.B. Tetrahydrofuran. Schließlich wird das so gereinigte Produkt getrocknet.

Das Herstellungsverfahren wird nachstehend anhand von Beispielen näher erläutert, wobei zu Vergleichszwecken auch die bekannten Platinkomplexverbindungen der Formeln II und III, in denen $R_1$ und $R_2$ Wasserstoffatome sind, hergestellt wurden.

In der nachfolgenden Zusammenstellung sind die als Ausgangssubstanz in den einzelnen Beispielen (Beispiele 1 und 7 als Vergleichsbeispiele) eingesetzten Naphthochinone der Formel IV aufgeführt:

| Beispiele Nr. | $R_1$ | $R_2$ | $R_3$ |
|---|---|---|---|
| 1, 7 | H | H | |
| 2, 8 | H | $CH_3$ | |
| 3, 9 | $CH_3$ | $CH_3$ | |
| 4, 10 | H | $-CH-CH_2-CH=C(CH_3)_2$<br>$OR_3$ | 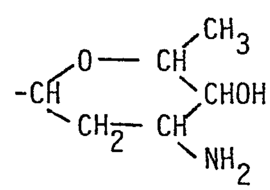 |
| 5, 11 | H | $-CH-CH_2-CH=C(CH_3)_2$<br>$OR_3$ | H |
| 6, 12 | H | $-CH-CH_2-CH=C(CH_3)_2$<br>$OR_3$ | O<br>$-C-C=CH-CH_3$<br>$CH_3$ |

Beispiele 1 bis 6: Herstellung von Platinkomplexverbindungen der Formel II

In allen Fällen wurde je 1 mMol des betreffenden Naphthochinons in 100 ml Ethanol gelöst und durch Zugabe wäßriger konzentrierter Ammoniaklösung auf einen pH-Wert von 8 eingestellt. Dieser ammoniakalischen alkoholischen Lösung wurde eine wäßrige Lösung von 2 mMol $K_2PtCl_4$ in 100 ml bidestilliertem Wasser unter ständigem Rühren bei Raumtemperatur zugefügt und das Gemisch über Nacht ebenfalls bei Raumtemperatur weitergerührt. Nach Einengen auf dem Rotovapor wurde die ausgeschiedene Platinkomplexverbindung über eine Fritte filtriert und mehrmals zuerst mit bidestilliertem Wasser, dann mit Aceton gewaschen und anschließend mit einem Ether (Tetrahydrofuran) in einem Soxhlet-Apparat von nicht umgesetztem Naphthochinonderivat befreit und schließlich im Vakuumexsikkator getrocknet.

Beispiele 7 bis 12: Herstellung von Platinkomplexverbindungen der Formel III

Die Arbeitsweise unterschied sich von der der Beispiele 1 bis 6 nur dadurch, daß vor der Zugabe der $K_2PtCl_4$-Lösung die alkoholische Lösung des Naphthochinons (IV) auf einen pH-Wert von 11 eingestellt wurde.

Alle in den Beispielen 1 bis 12 erhaltenen Platinkomplexverbindungen waren dunkle Pulver, die sich beim Versuch der Schmelzpunktbestimmung zersetzten und die in organischen Lösungsmitteln sehr schwer löslich bis unlöslich sind.

Die Elementaranalyse der erhaltenen Verbindungen ist in der nachstehenden Tabelle I zusammengestellt, in der zum Vergleich neben den gefundenen Analysenwerten auch die aus den jeweiligen Formeln errechneten Werte angegeben sind.

Tabelle I: Ergebnisse der Elementaranalysen

| Beispiel | | C | H | $NH_3$ | Cl | Pt |
|---|---|---|---|---|---|---|
| 1 | gef. | 17,92 | 0,64 | 6,61 | 10,4 | 56,0 |
| | ber. | 17,58 | 0,58 | 4,97 | 10,38 | 57,11 |
| 2 | gef. | 18,71 | 0,80 | 5,06 | 10,61 | 54,10 |
| | ber. | 18,95 | 0,86 | 4,87 | 10,17 | 55,95 |
| 3 | gef. | 16,92 | 1,26 | 4,91 | 10,20 | 55,12 |
| | ber. | 17,29 | 1,12 | 4,78 | 9,97 | 54,85 |
| 4 | gef. | 26,70 | 2,91 | 5,14 | 9,23 | 45,25 |
| | ber. | 28,39 | 2,70 | 3,82 | 7,98 | 43,92 |
| 5 | gef. | 25,22 | 1,94 | 4,60 | 10,13 | 48,16 |
| | ber. | 24,91 | 1,81 | 4,40 | 9,20 | 50,58 |
| 6 | gef. | 28,76 | 2,51 | 3,71 | 9,80 | 43,70 |
| | ber. | 29,21 | 2,33 | 3,93 | 8,20 | 45,18 |
| 7 | gef. | 16,31 | 0,61 | 4,73 | 19,40 | 52,00 |
| | ber. | 15,96 | 0,53 | 4,51 | 18,63 | 51,85 |
| 8 | gef. | 17,51 | 0,86 | 4,60 | 17,71 | 49,80 |
| | ber. | 17,23 | 0,78 | 4,43 | 18,29 | 50,90 |
| 9 | gef. | 18,10 | 1,10 | 4,71 | 18,25 | 50,40 |
| | ber. | 18,46 | 1,02 | 4,35 | 17,96 | 50,00 |
| 10 | gef. | 25,65 | 2,83 | 4,12 | 13,70 | 41,89 |
| | ber. | 26,04 | 2,47 | 3,51 | 14,64 | 40,28 |
| 11 | gef. | 21,81 | 1,92 | 4,40 | 15,50 | 48,16 |
| | ber. | 22,59 | 1,64 | 4,0 | 16,48 | 45,86 |
| 12 | gef. | 26,20 | 2,29 | 4,10 | 14,10 | 39,80 |
| | ber. | 27,0 | 2,14 | 3,64 | 15,0 | 41,75 |

## In vivo-Untersuchungen

Zur Ermittlung der antikarzinogenen Aktivität der erfindungsgemäßen Platinkomplexverbindungen wurde der Standardtest mit dem Ehrlich Ascites Tumor an ICR-Mäusen angewendet. Für die Experimente standen je eine Tiergruppe (10

Mäuse) pro eingesetzter Dosis des jeweiligen Platin-Naphthochinonderivat-Komplexes und je zwei Tiergruppen (20 Mäuse) für die Vergleichstests a) mit cis-DDP (positive Kontrolle) und b) ohne einen Antitumorwirkstoff (negative Kontrolle) zur Verfügung. Die Tumorzellen sowie die Platinkomplexverbindungen wurden am Tag Null bzw. am 1. und 6. Tag intraperitonal injiziert. Die Resultate wurden in der nachstehenden Tabelle II zusammengestellt.

Tabelle II: Antitumorwirkung von Platinkomplexverbindungen

| | Dosis mg Pt-Komplex pro kg | Anzahl der "Cures" pro Tiergruppe |
|---|---|---|
| Negative Kontrolle | 0 | 0 |
| Positive Kontrolle | 6 | 4 |
| Platin-Komplex aus Beispiel | | |
| 1 (Vergleich) | 20 | 7 |
| 2 | 20 | 8 |
| 3 | 20 | 7 |
| 4 | 30 | 8 |
| 5 | 30 | 8 |
| 6 | 30 | 9 |
| 7 (Vergleich) | 20 | 5 |
| 8 | 20 | 7 |
| 9 | 15 | 6 |
| 10 | 24 | 6 |
| 11 | 24 | 7 |
| 12 | 24 | 8 |

Der Terminus "Cure" wurde vom National Cancer Institute, USA, übernommen und bedeutet, wieviele Tiere länger als 60 Tage überlebt haben. Aus den Resultaten geht deutlich hervor, daß die erfindungsgemäßen Platinkomplexverbindungen gegenüber den bekannten Antitumormitteln der Vergleichsbeispiele 1 und 7 eine mindestens gleich gute, meist sogar deutlich bessere Antitumorwirkung zeigen.

## Patentansprüche

1. Platinkomplexverbindungen der allgemeinen Formel

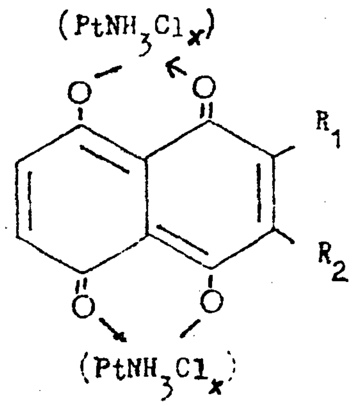

(I)

in der $R_1$ und $R_2$, die gleich oder verschieden sein können, einen Alkylrest mit 1 bis 18 C-Atomen oder $R_1$ oder $R_2$ ein Wasserstoffatom und $R_2$ oder $R_1$ einen Alkylrest mit 1 bis 18 C-Atomen oder einen Rest $-CH(OR_3)-CH_2-CH=C(CH_3)_2$ darstellen, wobei $R_3$ ein Wasserstoffatom oder den Rest 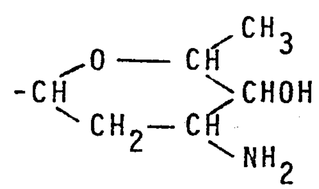 oder einen Acylrest bedeutet und x = 1 oder 2 ist.

2. Platinkomplexverbindungen nach Anspruch 1, dadurch gekennzeichnet, daß $R_1$ und $R_2$ Alkylreste mit 1 bis 6 C-Atomen bedeuten.

3. Platinkomplexverbindungen nach Anspruch 1 oder 2, dadurch gekennzeichnet, daß der Acylrest $R_3$ ein Angelikasäurerest ist.

4. Verfahren zur Herstellung von Platinkomplexverbindungen nach den Ansprüchen 1 bis 3, dadurch gekennzeichnet, daß man eine alkoholische Lösung eines 5,8-Dihydroxy-1,4-naphthochinons der Formel

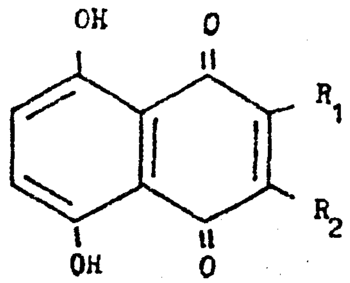

(IV)

in der die Substituenten $R_1$ und $R_2$ (einschließlich $R_3$) die vorstehend angegebenen Bedeutungen haben, durch Zugabe von konzentrierter wäßriger Ammoniaklösung auf einen pH-Wert über 7 einstellt und danach mit einer wäßrigen Lösung von $K_2PtCl_4$ bei Raumtemperatur umsetzt, wobei die Reaktionsmischung mehrere Stunden lang ständig gerührt wird, anschließend die ausgeschiedenen Platinkomplexverbindungen der allgemeinen Formel I abfiltriert und nacheinander mit Wasser, Aceton und gegebenenfalls einem Ether wäscht und schließlich trocknet.

5. Verfahren nach Anspruch 4, dadurch gekennzeichnet, daß die Umsetzung bei einem pH-Wert von 8 bis 8,5 durchgeführt wird, wobei im wesentlichen Platinkomplexverbindungen der Formel I mit x = 1 erhalten werden.

6. Verfahren nach Anspruch 4, dadurch gekennzeichnet, daß die Umsetzung bei einem pH-Wert von über 10 erfolgt, wobei im wesentlichen Platinkomplexverbindungen der Formel I mit x = 2 erhalten werden.

7. Verwendung der Platinkomplexverbindungen gemäß Ansprüchen 1 bis 3 als Wirkstoffe mit Antitumorwirkung.